(19) Europäisches Patentamt
European Patent Office
Office européen des brevets

(11) EP 0 876 329 B1

(12) EUROPÄISCHE PATENTSCHRIFT

(45) Veröffentlichungstag und Bekanntmachung des Hinweises auf die Patenterteilung:
**18.07.2001 Patentblatt 2001/29**

(21) Anmeldenummer: **96943039.6**

(22) Anmeldetag: **05.12.1996**

(51) Int Cl.[7]: **C07C 231/02**, C07C 233/47, C07C 323/59

(86) Internationale Anmeldenummer:
**PCT/EP96/05439**

(87) Internationale Veröffentlichungsnummer:
**WO 97/21667 (19.06.1997 Gazette 1997/26)**

(54) **VERFAHREN ZUR HERSTELLUNG VON N-ACETYL-D,L-ALPHA-AMINOCARBONSÄUREN**

PROCESS FOR THE PRODUCTION OF N-ACETYL-D,L-ALPHA-AMINOCARBOXYLIC ACIDS

PROCEDE DE PRODUCTION DE N-ACETYL-D,L-ALPHA-AMINOACIDES

(84) Benannte Vertragsstaaten:
**AT BE CH DE DK ES FI FR GB IE IT LI NL PT SE**

(30) Priorität: **13.12.1995 DE 19546533**

(43) Veröffentlichungstag der Anmeldung:
**11.11.1998 Patentblatt 1998/46**

(73) Patentinhaber: **Degussa AG**
**40474 Düsseldorf (DE)**

(72) Erfinder:
- **DRAUZ, Karlheinz**
  **D-63579 Freigericht (DE)**
- **BOMMARIUS, Andreas**
  **D-60596 Frankfurt (DE)**
- **KARRENBAUER, Michael**
  **D-78345 Moos (DE)**
- **KNAUP, Günter**
  **D-63486 Bruchköbel (DE)**

(56) Entgegenhaltungen:
- **J. BIOL. CHEM., Bd. 178, 1949, Seiten 503-9, XP000645688 P. J. FODOR ET AL.: "Preparation of L- and D-Alanine by enzymatic resolution od acetyl-DL-alanine"**
- **Z. PHYSIOL. CHEM., Bd. 146, 1925, Seiten 267-275, XP000646153 F. KNOOP ET AL.: "Über die Acetylierung von Aminosäuren im Tierkörper"**
- **DATABASE WPI Section Ch, Week 8115 Derwent Publications Ltd., London, GB; Class B05, AN 81-26924D XP002027284 & SU 753 844 A (BIOKHIMREAKTIV RES) , 11.August 1980**

EP 0 876 329 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung bezieht sich auf ein Verfahren zur Herstellung von N-Acetyl-D,L-α-aminocarbonsäuren aus den entsprechenden D,L-α-Aminocarbonsäuren durch Acetylierung mit Acetanhydrid in Essigsäure und Isolierung der resultierenden N-Acetyl-D,L-α-aminocarbonsäuren aus dem Acetylierungsgemisch.

N-Acetyl-D,L-α-aminocarbonsäuren sind u. a. als Zwischenprodukte in Verfahren zur Herstellung optisch aktiver α-Aminosäuren aus entsprechenden racemischen D,L-α-Aminocarbonsäuren von Interesse.

Diese Verfahren können sich im wesentlichen und insbesondere aus folgenden Teilschritten zusammensetzen:

1) Herstellung eines racemischen Gemisches der N-Acetyl-D,L-α-aminocarbonsäuren aus den racemischen D,L-α-Aminocarbonsäuren;

2) Enzymatische Spaltung der im racemischen Gemisch enthaltenen N-Acetyl-L-α-aminocarbonsäuren;

3) Trennung der L-α-Aminocarbonsäuren und der N-Acetyl-D(L)-α-aminocarbonsäuren; und

4) Racemisierung und Rückführung der nicht umgesetzten N-Acetyl -D-α-aminocarbonsäuren.

Das gebräuchlichste Verfahren zur Acetylierung von Aminosäuren ist die Schotten-Baumann-Acetylierung. Hierzu wird eine wäßrige Lösung der Aminosäuren unter basischen Bedingungen mit Acetanhydrid oder Acetylchlorid versetzt. Die Isolierung der Acetylaminosäuren erfolgt nach Ansäuern durch Filtration oder Extraktion.

Nachteilig an diesem Verfahren ist, daß bedingt durch die im wäßrigen Medium ablaufende Hydrolyse stets ein größerer Überschuß an Acetylierungsreagenz eingesetzt werden muß und daß pro Mol Acetylaminosäuren mindestens zwei Mol unerwünschtes Salz entstehen.

In J. Biol. Chem 1949, 1778,503-9 wird die Herstellung von N-Acetyl-D,L-Alanin mit 1,5 Äquivalenten Acetanhydrid beschrieben. Die SU-754844 offenbart ein ebensolches Verfahren in Bezug auf D,L-Valin.

In der SU-A-1 293 171 wird ein Verfahren zur Acetylierung von Alanin beschrieben, bei dem D,L-Alanin mit 1,2 bis 1,3 Mol-äquivalenten Acetanhydrid in 4,4 bis 5,0 Mol-äquivalenten Essigsäure bei 48 bis 55 °C acetyliert wird. D. h. gemäß SU-A-1 293 171 wird in einer Konzentration von ca. 3,3 - 3,8 Mol Alanin pro 1 1 Essigsäure acetyliert. Nach Abdampfen der Essigsäure unter vermindertem Druck, Kristallisation aus Wasser und Trocknung werden 78 bis 83 % Acetyl-D,L-alanin in einer Reinheit von 98,7 bis 99 % erhalten.

Damit wird zwar eine "salzfreie" Acetylierungsvariante offenbart; nachteilig an diesem Verfahren ist jedoch die Notwendigkeit der wässrigen Aufarbeitung zur Gewinnung der N-acetylierten α-Aminosäuren in ausreichender Reinheit. Insbesondere die Kristallisation aus Wasser ist relativ umständlich und aufwendig, und steht bis jetzt einer technischen Realisierung des Verfahrens hindernd entgegen.

In Anbetracht des vorstehend angeführten und diskutierten Standes der Technik liegt der Erfindung die Aufgabe zugrunde ein weiteres Verfahren zur Herstellung von N-Acetyl-D,L-α-aminocarbonsäuren anzugeben, das die im berücksichtigten Stand der Technik vorhandenen Schwierigkeiten überwindet, also insbesondere "salzfrei", das heißt ohne den Zwangsanfall von Salz, die einfache Gewinnung von N-Acetyl-D,L-α-aminocarbonsäuren in hoher Reinheit möglichst ohne wäßrige Aufarbeitung gestattet.

Erfüllt werden die genannten Anforderungen von einem Verfahren der eingangs erwähnten Gattung mit den Merkmalen des kennzeichnenden Teils des Anspruchs 1. Weitere vorteilhafte Ausgestaltungen der Erfindung werden in den von Anspruch 1 abhängigen Ansprüchen unter Schutz gestellt.

Dadurch, daß die Acetylierung mit 1,0 - 1,1 Mol Acetanhydrid pro Mol zu acetylierender D,L-α-Aminosäure bei 60 - 150 °C in einer Konzentration von < 3 Mol α-Aminocarbonsäure pro 1 Liter Essigsäure durchgeführt wird, wobei die Konzentration bei Standardbedingungen von Druck und Temperatur bestimmt wird, und die N-Acetyl-D,L-α -Aminocarbonsäure durch Eindampfen als Schmelze oder Feststoff erhalten wird, kann vollständig auf die wäßrige Aufarbeitung der N-Acetyl-D,L-α-aminocarbonsäuren verzichtet werden, wobei dennoch eine Qualität der Acetylaminocarbonsäuren erhalten wird, die so hoch ist, daß beispielsweise deren direkter Einsatz in der enzymatischen Spaltung eines Verfahrens zur Herstellung optisch aktiver α-Aminocarbonsäuren möglich ist.

Insbesondere und überraschenderweise wurde nun gefunden, daß das erfindungsgemäße Verfahren bevorzugt so durchgeführt werden kann, daß die angestrebten Produkte ohne wäßrige Aufarbeitung mit Reinheiten von > 95 % bei gleichzeitig sehr hohen Ausbeuten erhalten werden können.

Im Gegensatz zu dem aus der SU 1 293 171 bekannten Verfahren ist es dabei als besonders bemerkenswert zu bewerten, daß eine höhere Temperatur bei der Acetylierung in Verbindung mit einer Verwendung einer höheren Menge Essigsäure als Lösungsmittel bei mindestens gleich hoher Ausbeute zu einer deutlich höheren Reinheit der acetylierten Zielprodukte führt.

Ein wichtiger erfindungsgemäßer Aspekt besteht in der Erhöhung der Acetylierungstemperatur auf 60 - 150 °C. Dabei hängt die zu wählende Temperatur in gewissem Maße auch von der zu acetylierenden Aminosäure ab. In bevorzugter Abwandlung des Verfahrens der Erfindung wird die Acetylierung bei Temperaturen zwischen 85 und 115 °C vorgenommen.

Neben der Temperatur der Acetylierungsreaktion ist ein zweiter wesentlicher Aspekt der Erfindung im Verhältnis von umzusetzender Aminocarbonsäure und Lösungsmittel Essigsäure zu sehen. Durch eine verdünntere Fahrweise gelang es in nicht ohne weiteres vorhersehbarer Weise, die Bildung von Nebenprodukten zu verringern.

In weiterhin bevorzugter Ausführungsform ist das Verfahren der Erfindung dadurch gekennzeichnet, daß die Konzentration der zu acetylierenden α-Aminocarbonsäuren pro 1 Liter Essigsäure zwischen 0,5 und 2,5 Mol beträgt, wobei die Konzentration auf Standardbedingungen von Druck und Temperatur bezogen ist.

Nachdem gemäß dem Stand der Technik weiterhin ein Überschuß von 0,2 - 0,3 Moläquivalenten Acetylierungsmittel (Acetanhydrid) auf die stöchiometrisch notwendige Menge als essentiell betrachtet wird, hat sich im Rahmen der Erfindung doch einigermaßen unerwartet herausgestellt, daß eine vollständige Acetylierung bereits mit äquimolaren Mengen Acetanhydrid erfolgt. Um jedoch einen vollständigen Umsatz sicherzustellen, ist ein 5 - 10 %iger Überschuß empfehlenswert.

Erfindungsgemäß ist daher ein Verfahren, bei dem man pro Mol zu acetylierender D,L-α-Aminosäure 1,0 bis 1,1 Mol Acetanhydrid einsetzt.

Schließlich besteht ein weiterer wesentlicher Aspekt darin, daß die N-Acetyl-D,L-α-aminocarbonsäuren durch Eindampfen als Schmelze oder Feststoff direkt erhalten werden, d. h., insbesondere ohne wäßrige Aufarbeitung, was besonders vorteilhaft ist, und dabei in hoher Reinheit, wie bereits erwähnt.

Daher kennzeichnet sich das Verfahren der Erfindung in vorteilhafter Variante dadurch, daß die N-Acetyl-D, L-α-aminocarbonsäuren durch Eindampfen ohne weitere Isolierung oder Aufreinigung in einer Reinheit von > 95 % (bezogen auf das Gewicht) erhalten werden.

Bei der Nacharbeitung der SU 1 293 171 wurde festgestellt, daß unter den in der SU 1 293 171 beschriebenen Bedingungen in einem erheblichen Ausmaß ein Nebenprodukt gebildet wird, das als Acetyldipeptid identifiziert werden konnte. Bei Durchführung der Reaktion kann die Bildung der AcetylDipeptide erfindungsgemäß auf Werte unter 5 %, unter optimalen Bedingungen sogar unter 2 %, reduziert werden.

Es ist demnach erfindungsgemäß bevorzugt, daß N-acetylierte D,L-α-Aminocarbonsäuren erhalten werden, die weniger als 5 Gew.-% an Acetyldipeptiden als Nebenprodukt enthalten, bezogen auf 100 Gew.-% des durch Eindampfen als Schmelze oder Feststoff erhaltenen Produkts.

Es ist ferner besonders bevorzugt, daß ein Produkt erhalten wird, das weniger als 2 Gew.-% Acetyldipeptide aufweist.

Derart an Nebenprodukte arme N-Acetyl-D,L-α-aminocarbonsäuren sind besonders wertvolle Produkte und Zwischenprodukte.

Auf diesem Weg hergestellte Acetylaminosäuren können ohne weitere Reinigung als Zwischenstufe in verschiedenen Verfahren eingesetzt werden. Die Isolierung der Acetylaminosäuren kann dadurch erfolgen, daß die Essigsäurelösung bis zur Schmelze bzw. bis zum Feststoff eingeengt wird. Als geeignete Geräte hierfür können z. B. Sambay-Verdampfer eingesetzt werden. Die abdestillierte Essigsäure kann, da wasserfrei, direkt beim Folgeansatz wieder eingesetzt werden.

Das erfindungsgemäße Acetylierungsverfahren ist auf eine Vielzahl von α-Aminocarbonsäuren anwendbar. Zu den erfolgreich zu acetylierenden Verbindungen gehören u. a. Verbindungen der Formel I

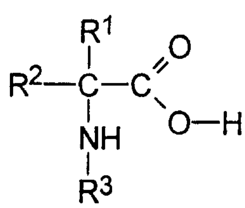

I,

worin

$R^1$ Wasserstoff oder $C_{1-4}$-Alkyl,

$R^2$ Wasserstoff, Alkyl, substituiertes Alkyl, Aryl, substituiertes Aryl, Heteroaryl, Arylalkyl, Heteroarylalkyl, Cycloalkyl- oder Cycloalkylalkyl ist, wobei die genannten Reste jeweils wiederum selbst substituiert sein können und/oder Heteroatome enthalten können,
oder

$R^1$ und $R^2$ zusammen mit dem sie verbindenden C-Atom einen 3 bis 7-gliedrigen gesättigten Ring bilden,

$R^3$ Wasserstoff oder $C_{1-4}$-Alkyl ist

oder

$R^2$ und $R^3$ zusammen mit dem sie verbindenden N- und C-Atom einen 4 bis 7-gliedrigen gesättigten Ring bilden, der ggf. Heteroatome enthalten kann.

Alkylgruppen können geradkettig oder verzweigt sein, bevorzugt sind Kettenlängen von $C_1$-$C_{12}$ bei geraden Ketten bzw. Kettenlängen von $C_{3-12}$ bei verzweigten Ketten, besonders bevorzugt Kettenlängen von $C_{1-6}$ bei geraden Ketten bzw. $C_{3-6}$ bei verzweigten Ketten, Beispiele sind Methyl, Ethyl, Propyl, Isopropyl, Butyl, Isooctyl, Dodecyl.

Diese Alkylgruppen sind bevorzugt substituiert durch 1 - 3 Amino-, Hydroxyl-, Halogen-, Guanidino-, Harnstoff-, Carboxy-, Carboxamid- und/oder Alkoxycarbonyl-Gruppen.

Arylgruppen können bevorzugt Phenyl- oder substituierte Phenyl-Gruppen sein.

Substituierte Arylgruppen sind bevorzugt Mono-, Di-, oder Trihalogen, Mono-, Di-, oder Trihydroxy, Mono-, Di-, Trialkyl-Phenylgruppen, wobei Halogen Fluor Chlor oder Brom ist, Alkyl $C_{1-4}$-Alkyl, bevorzugt Methyl oder Ethyl.

Heteroaryl-Gruppen sind bevorzugt 5 oder 6-Ring-Systeme mit 1 - 2 Heteroatomen im Ring, die O, N, oder S sein können.

Arylalkyl ist bevorzugt Benzyl. Cycloalkyl und Cycloalkylmethyl sind bevorzugt $C_{3-7}$-Ring-Systeme.

Bevorzugt sind Alanin, Valin, Leucin, Isoleucin, Prolin, Tryptophan, Phenylalanin, Methionin, Serin, Tyrosin, Threonin, Cystein, Asparagin, Glutamin, Histidin, Cystin, Citrullin, Homocystein, Homoserin, Hydroxyprolin, Ornithin, Norvalin sowie Derivate der vorstehend genannten Aminosäuren. Ganz besonders bevorzugt wird D,L-Methionin acetyliert.

Das erfindungsgemäße Verfahren ist grundsätzlich zur N-Acetylierung von α-Aminocarbonsäuren geeignet, gleichgültig welchen Ursprungs. Die Acetylierung läßt sich dabei hervorragend in einen Prozeß zur Gewinnung optisch aktiver α-Aminosäuren integrieren.

Dabei kennzeichnet sich ein solches integriertes Verfahren zur Gewinnung von z. B. L-Methionin dadurch, daß die Acetylierung des D,L-Methionin eine Stufe eines Verfahrens zur Gewinnung optisch aktiver α-Aminosäuren ist, bei dem D,L-Methionin zunächst mit Essigsäure/Acetanhydrid N-acetyliert wird, das N-Acetyl-D,L-Methionin-Racemat enzymatisch gespalten wird, das L-Methionin unter Rückbehalt einer Mutterlauge abgetrennt wird und das nicht umgesetzte N-Acetyl-D(L)-Methionin der Mutterlauge nach Racemisierung als Rückführlösung zur enzymatischen Racemat-Spaltung rezykliert wird.

Das bei dem derart modifizierten Verfahren eingesetzte N-Acetyl-D(L)-Methioninnatriumsalz und N-Acetyl-D (L)-Methionin kann dabei zweckmäßigerweise so gewonnen werden, daß man die bei der enzymatischen Hydrolyse nach erfolgter Isolierung des L-Methionins anfallende Mutterlauge über einen stark sauren Kationenaustauscher schickt, der die enthaltenen Kationen und die restliche L-α-Aminocarbonsäure adsorbiert. Die aus dem Ionenaustauscher austretende Lösung besteht dann praktisch nur noch aus Wasser, Essigsäure und N-Acetyl-D(L)-Methionin. Sie wird beispielsweise unter schonenden Bedingungen bei niedrigen Temperaturen im Vakuum, beispielsweise in einer Kombination aus Fallfilmverdampfer und Dünnschichtverdampfer mit Feststoffaustrag zwecks Entfernung der die enzymatische Umsetzung inhibierenden Essigsäure eingedampft und in wäßrige Natronlauge bei pH 4 - 8, vorzugsweise 4,5 - 5,5, eingetragen. Alternativ dazu kann die NaOH auch bereits vor Entfernung der Essigsäure zugesetzt werden. Dadurch wird der pH-Wert schon vor der Eindampfung erhöht, wodurch die Hydrolyse, im gewählten Beispiel von N-Ac-D(L)-Methionin, reduziert werden kann.

Auch die zum Aufschmelzen der N-Acetyl-D(L)-α-aminocarbonsäure erforderliche Verweilzeit soll zweckmäßigerweise möglichst kurz gehalten werden. Nimmt man das Aufschmelzen in einem beheizten Extruder vor, so reicht für das vollständige Aufschmelzen im allgemeinen eine Verweilzeit von weniger als einer Minute. In diesem Fall kann der Extruder die Schmelze in ein beheiztes Reaktionsrohr fördern, wo zu Beginn der Verweilstrecke eine entsprechend ausgelegte Pumpe kontinuierlich die erforderliche Menge an Essigsäureanhydrid über ein Mischsystem zudosiert.

Die Erfindung wird durch die nachfolgenden Beispiele näher erläutert. Alle Prozentangaben bedeuten, wenn nicht anders angegeben, Gewichtsprozente.

Beispiel 1: Herstellung von N-Acetyl-D,L-methionin

Unterschiedliche Mengen D,L-Methionin wurden in 100 ml Essigsäure gelöst, die Lösung im Ölbad temperiert und 1.1 Äquivalente Acetanhydrid zugegeben. Nach 5 min wurden die Ansätze im Vakuum bei 100 °C Badtemperatur eingeengt. Der Gehalt an N-Acetyl-D,L-methionyl-D,L-methionin (Ac-Met-Met) wurde per HPLC bestimmt. Die Ergebnisse dieser Versuche sind in der folgenden Tabelle zusammengefaßt:

| D,L-Methionin | Essigsäure | Acetanhydrid | Temp. | Ac-Met-Met |
|---|---|---|---|---|
| 7.5 g (50 mmol) | 100 ml | 5.6 g (55 mmol) | 80 °C | 1.8 % |
| 7.5 g (50 mmol) | 100 ml | 5.6 g (55 mmol) | 95 °C | 1.8 % |
| 14.9 g (100 mmol) | 100 ml | 11.2 g (110 mmol) | 95 °C | 3.2 % |
| 26.1 g (175 mmol) | 100 ml | 19.6 g (192 mmol) | 95 °C | 5.9 % |
| 37.3 g (250 mmol) | 100 ml | 28.1 g (275 mmol) | 95 °C | 7.8 % |

Beispiel 2: Herstellung von N-Acetyl-D,L-methionin unter technischen Bedingungen

373 g (2.5 mol) D,L-Methionin wurden in 5 1 Essigsäure bei 90 °C gelöst und mit 281 g (2.75 mol) Acetanhydrid versetzt. Die Temperatur stieg dabei auf 95 °C an. Nach 10 min Nachreaktion wurde die Lösung an einem Sambay-Verdampfer bei 170 °C und 9 mbar eingeengt. Man erhielt 427 g eines zähen Öls, das beim Erkalten erstarrte. Nach HPLC-Analyse bestand das Produkt aus 92.3 % Acetyl-D,L-Methionin und 3.4 % Ac-Met-Met.

Beispiel 3: Herstellung von N-Acetyl-D,L-valin

11.7 g (0.10 mol) D,L-Valin wurden in 100 ml Essigsäure gelöst und bei 90 °C mit 11.2 g (0.11 mol) Acetanhydrid versetzt. Nach 10 min wurde bei 15 mbar und 100 °C eingeengt. Nach HPLC-Analyse bestand der kristalline Rückstand aus 95.6 % Acetyl-D,L-valin und 2.8 % N-Acetyl-D,L-valyl-D,L-valin.

Beispiel 4: Herstellung von N-Acetyl-D,L-Norvalin

11.7 g (0.10 mol) D,L-Norvalin wurden analog Beispiel 3 umgesetzt. Der kristalline Rückstand bestand nach HPLC-Analyse aus 95.2 % N-Acetyl-D,L-norvalin und 4.1 % N-Acetyl-D,L-norvalyl-D,L-norvalin.

Beispiel 5: Entfernen von Essigsäure aus einer Mischung von aus N-Acetyl-D,L-methionin und Natriumacetat

Jeweils 38.2 g (0.20 mol) N-Acetyl-D,L-methionin und 16.4 g (0.20 mol) Natriumacetat wurden intensiv gemischt und erhitzt. Ab ca. 100 °C bildeten sich Schmelzen. Die Schmelze wurde bei 15 mbar 30 min auf 150 - 180 °C erhitzt und nach Abkühlen der Gehalt an Essigsäure bestimmt. Die Ergebnisse sind in der folgenden Tabelle zusammengefaßt:

| Temperatur | Essigsäure |
|---|---|
| 150 °C | 5.3 % |
| 160 °C | 4.3 % |
| 170 °C | 3.0 % |
| 180 °C | 3.0 % |

Beispiel 6: Entfernen von Essigsäure aus einer Mischung von aus N-Acetyl-L-valin und Natriumacetat

79.6 g (0.50 mol) L-Valin und 41.0 g (0.5 mol) Natriumacetat wurden unter Erwärmen in 150 ml Wasser gelöst und bei 100 °C und 15 mbar zur Trockne eingeengt. Teile des festen Rückstandes wurden anschließend für jeweils 30 min bei verschiedenen Temperaturen getrocknet und der Gehalt an Essigsäure bestimmt. Die Ergebnisse sind in der folgenden Tabelle zusammengefaßt:

| Temperatur | Essigsäure |
|---|---|
| 100 °C | 10.4 % |
| 150 °C | 5.5 % |
| 175 °C | 2.1 % |
| 200 °C | 1.7 % |

Beispiel 7: Entfernen von Essigsäure aus einer Mischung von aus N-Acetyl-L-phenylalanin und Natriumacetat

103.6 g (0.50 mol) L-Phenylalanin und 41.0 g (0.5 mol) Natriumacetat wurden unter Erwärmen in 150 ml Wasser gelöst und bei 100 °C und 15 mbar zur Trockne eingeengt. Teile des festen Rückstandes wurden anschließend für jeweils 30 min bei verschiedenen Temperaturen getrocknet und der Gehalt an Essigsäure bestimmt. Die Ergebnisse sind in der folgenden Tabelle zusammengefaßt:

| Temperatur | Essigsäure |
|---|---|
| 100 °C | 7.3 % |
| 150 °C | 3.5 % |
| 175 °C | < 0.5 % |

Beispiel 8: Entfernen von Essigsäure aus einer Mischung von aus N-Acetyl-D,L-Norvalin und Natriumacetat

Jeweils 3.12 g (0.02 mol) N-Acetyl-D,L-Norvalin und 1.64 g (0.02 mol) Natriumacetat wurden intensiv gemischt und erhitzt. Ab ca. 110 °C bildete sich eine Schmelze. Die Schmelze wurde bei 15 mbar 30 min auf 160 °C erhitzt. Nach dem Abkühlen enthielt die Probe 2.2 % Essigsäure.

Beispiel 9: Vergleich der thermischen Beständigkeit von N-Acetyl-D,L-methionin und N-Acetyl-D,L-methionin-natriumsalz

Jeweils 20 g N-Acetyl-D,L-methionin und N-Acetyl-D,L-methionin-natriumsalz wurden auf 150 °C erhitzt. Die Bildung von N-Acetyl-D,L-methionyl-D,L-methionin wurde mit Hilfe der HPLC verfolgt. Die Ergebnisse sind in der folgenden Tabelle zusammengefaßt:

| Zeit | Ac-Met-OH | Ac-Met-ONa |
|---|---|---|
| 1 h | 1.7 % | 0.1 % |
| 2 h | 3.8 % | 0.2 % |
| 5 h | 8.8 % | 0.4 % |

Beispiel 10: Racemisierung von N-Acetyl-D(L)-methioninnatrium in der Schmelze

Jeweils 10 g (0.053 mol) N-Acetyl-D(L)-methionin wurden mit 8 gew.-%iger Natronlauge auf unterschiedliche pH-Werte gestellt und im Vakuum bei 50 mbar bis zur Schmelze eingedampft. Anschließend wurden die Schmelzen erhitzt und unter guter Rührung Essigsäureanhydrid zudosiert. Diese Schmelze wurde dann für eine bestimmte Zeit bei dieser Temperatur belassen und anschließend in Wasser aufgenommen. Zur Verfolgung der Racemisierung wurde der Drehwert ($[\alpha]_D^{20}$, c = 1 in Wasser) bestimmt. Vor der Racemisierung betrug dieser Drehwert +21.3. Die Ergebnisse dieser Versuche sind in der folgenden Tabelle zusammengefaßt:

| pH-Wert | $AC_2O$ | Temp. | Verweilzeit | $[\alpha]_D^{20}$ | Ac-Met-Met |
|---|---|---|---|---|---|
| 5 | 2 % | 155 °C | 30 min | 0 | 0.3 % |
| 4 | 2 % | 155 °C | 30 min | +0.2 | 0.8 % |
| 6 | 2 % | 155 °C | 30 min | 0 | 0.2 % |
| 5 | 3 % | 110 - 120 °C | 30 min | +0.5 | 0.05 % |
| 5 | 2 % | 180 °C | 10 min | 0 | 0.1 % |
| 5 | 2 % | 180 °C | 30 min | 0 | 0.8 % |
| 8 | 3 % | 155 °C | 30 min | 0 | 0.4 % |
| 5 | 6 % | 155 °C | 30 min | 0 | 0.5 % |
| 1.6 | 2 % | 155 °C | 30 min | 0 | 5.5 % |

Beispiel 11: Racemisierung von N-Acetyl-L-phenylalaninnatriumsalz

Entsprechend Beispiel 7 bereitete und bei 150 °C im Vakuum getrocknete Proben von N-Acetyl-L-phenylalaninnatriumsalzen wurden mit Actanhydrid vermischt und erwärmt. Zur Verfolgung der Racemisierung wurde der Drehwert ($[\alpha]_D^{20}$, c = 1 in 1 N HCl) bestimmt. Vor der Racemisierung betrug dieser Drehwert +21.6. Die Ergebnisse sind in der folgenden Tabelle zusammengefaßt:

| $AC_2O$ | Temp. | Verweilzeit | $[\alpha]_D^{20}$ |
|---|---|---|---|
| 2 % | 140 °C | 15 min | 9.0 |
| 2 % | 160 °C | 15 min | 2.9 |
| 2 % | 180 °C | 15 min | 1.9 |
| 5 % | 160 °C | 15 min | 0 |
| - | 230 °C | 15 min | 10.8 |

Beispiel 12: Racemisierung von N-Acetyl-L-valin-natriumsalz

Entsprechend Beispiel 6 bereitete und bei 150 °C im Vakuum getrocknete Proben von N-Acetyl-L-valin-natriumsalzen wurden mit Acetanhydrid vermischt und erwärmt. Zur Verfolgung der Racemisierung wurde der Drehwert ($[\alpha]_D^{20}$, c = 1 in 1 N HCl) bestimmt. Vor der Racemisierung betrug dieser Drehwert +11.9. Die Ergebnisse sind in der folgenden Tabelle zusammengefaßt:

| $Ac_2O$ | Temp. | Verweilzeit | $[\alpha]_D^{20}$ |
|---|---|---|---|
| 2 % | 140 °C | 15 min | 10.2 |
| 2 % | 160 °C | 15 min | 6.5 |
| 2 % | 180 °C | 15 min | 4.8 |
| 5 % | 160 °C | 15 min | 1.6 |
| 5 % | 230 °C | 15 min | 0 |
| - | 230 °C | 15 min | 8.3 |

Beispiel 13: Nachacetylierung einer N-Acetyl-D(L)-methionin-Mutterlauge

Je 100 g einer nach Abtrennung von L-Methionin durch Filtration erhaltenen Mutterlauge, die pro 100 g 8.3 g (0.100 mol) Natriumacetat, 19.3 g (0.089 mol) N-Acetyl-D(L)-methionin-natrium und 1.7 g (0.011 mol) L-Methionin enthielt, wurde bei verschiedenen Temperaturen mit unterschiedlichen Mengen Acetanhydrid versetzt. Nach jeweils 30 min wurden Proben entnommen und der Methioningehalt mit Hilfe der HPLC bestimmt. Die Ergebnisse sind in der folgenden Tabelle zusammengefaßt:

| Temp. | Acetanhydrid | Methionin |
|---|---|---|
| 30 °C | - | 9.0 F1.-% |
| 30 °C | 1.16 g (11.4 mmol) | 2.9 F1.-% |
| 30 °C | 1.74 g (17.1 mmol) | 1.2 F1.-% |
| 30 °C | 2.03 g (19.9 mmol) | 0.6 Fl.-% |
| 30 °C | 2.61 g (25.6 mmol) | 0.2 Fl.-% |
| 60 °C | 1.16 g (11.4 mmol) | 2.9 Fl.-% |
| 60 °C | 1.74 g (17.1 mmol) | 0.2 Fl.-% |
| 60 °C | 2.03 g (19.9 mmol) | 0.0 Fl.-% |
| 90 °C | 1.16 g (11.4 mmol) | 2.9 Fl.-% |

(fortgesetzt)

| Temp. | Acetanhydrid | Methionin |
|---|---|---|
| 90 °C | 1.74 g (17.1 mmol) | 0.3 Fl.-% |
| 90 °C | 2.03 g (19.9 mmol) | 0.1 Fl.-% |

Beispiel 14: Recyclierung einer N-Acetyl-D(L)-Methionin-natriumsalz-Mutterlauge

717 g (3.75 mol) N-Acetyl-D,L-methionin und 140 g (3.50 mol) Natriumhydroxid wurden in Wasser gelöst und auf 1.5 1 aufgefüllt. Der pH-Wert der Lösung wurde mit 50 %iger Natronlauge auf 7 gestellt. Nach Zugabe von 3.6 g Acylase (Aktivität 31.000 E/g) wurde 5 Tage bei Raumtemperatur gerührt. Die Suspension wurde auf 5 °C abgekühlt, der Niederschlag abfiltriert, mit 300 ml Eiswasser gewaschen und getrocknet. Man erhielt 174 g (1.17 mol) L-Methionin. Das Filtrat (1800 g), das neben 21.8 Gew.-% N-Acetyl-D(L)-methionin 2.8 Gew.-% L-Methionin enthielt, wurde auf 60 °C erwärmt und mit 60 g (0.60 mol) Acetanhydrid versetzt. Nach 30 min war in der Lösung kein Methionin nachweisbar. Es wurden 223 g (1.17 mol) N-Acetyl-D,L-methionin zugegeben und im Vakuum auf 1200 g eingeengt. Diese Lösung wurde mit einem Sambay-Verdampfer bei 170 °C und 10 mbar auf 752 g eingeengt. In die noch warme Schmelze wurden 15 g Acetanhydrid eingerührt, 10 min auf 140 °C erhitzt und dann in Wasser gelöst und auf 1.5 1 aufgefüllt. Der Drehwert der Lösung war Null. Der Gehalt an N-Acetyldipeptid betrug 0.8 % bez. auf N-Acetyl-methionin. Nachdem der pH-Wert mit Natronlauge auf 7 gestellt wurde, wurde diese Lösung wie oben beschrieben erneut zur Acylasespaltung eingesetzt. Man erhielt 166 g (1.11 mol) L-Methionin.

Beispiel 15: Recyclierung einer N-Acetyl-D(L)-Norvalin-natriumsalz-Mutterlauge

597 g (3.75 mol) N-Acetyl-D,L-norvalin und 140 g (3.50 mol) Natriumhydroxid wurden in Wasser gelöst und auf 1.5 1 aufgefüllt. Der pH-Wert der Lösung wurde mit 50 %iger Natronlauge auf 7 gestellt. Nach Zugabe von 3.0 g Acylase (Aktivität 31.000 E/g) wurde 5 Tage bei Raumtemperatur gerührt. Die Suspension wurde auf 5 °C abgekühlt, der Niederschlag abfiltriert, mit 300 ml Eiswasser gewaschen und getrocknet. Man erhielt 157 g (1.34 mol) L-Norvalin. Das Filtrat (1393 g), das neben 22.5 Gew.-% N-Acetyl-D(L)-norvalin 3.1 Gew.-% L-Norvalin enthielt, wurde auf 60 °C erwärmt und mit 64 g (0.62 mol) Acetanhydrid versetzt. Nach 30 min war in der Lösung kein Norvalin nachweisbar. Es wurden 213 g (1.34 mol) N-Acetyl-D,L-norvalin zugegeben und im Vakuum auf 950 g eingeengt. Diese Lösung wurde mit einem Sambay-Verdampfer bei 170 °C und 10 mbar auf 644 g eingeengt. In die noch warme Schmelze wurden 13 g Acetanhydrid eingerührt, 10 min auf 140 °C erhitzt und dann in Wasser gelöst und auf 1.5 1 aufgefüllt. Eine chromatographische ee-Bestimmung erbrachte ein Verhältnis D/L von 51.3 % zu 48.7 %. Der Gehalt an N-Acetyldipeptid betrug 1.2 % bez. auf N-Acetyl-norvalin. Nachdem der pH-Wert mit Natronlauge auf 7 gestellt wurde, wurde diese Lösung wie oben beschrieben erneut zur Acylasespaltung eingesetzt. Man erhielt 113 g (0.97 mol) L-Norvalin.

Weitere Ausführungsformen der Erfindung ergeben sich aus den nachfolgenden Patentansprüchen.

## Patentansprüche

1. Verfahren zur Herstellung von N-Acetyl-D,L-α-aminocarbonsäuren aus den entsprechenden D,L-α-Aminocarbonsäuren durch Acetylierung in Essigsäure und Isolierung der resultierenden N-Acetyl-D,L-α-aminocarbonsäuren aus dem Acetylierungsgemisch,
   **dadurch gekennzeichnet**,
   daß die Acetylierung mit 1,0 - 1,1 Mol Acetanhydrid pro Mol zu acetylierender D,L-α-Aminosäure bei 60 - 150 °C in einer Konzentration von < 3 Mol α-Aminocarbonsäure pro 1 1 Essigsäure durchgeführt wird, wobei die Konzentration auf Standardbedingungen von Druck und Temperatur bezogen ist,
   und die N-Acetyl-D,L-α-aminocarbonsäure durch Eindampfen als Schmelze oder Feststoff erhalten wird.

2. Verfahren nach Anspruch 1,
   **dadurch gekennzeichnet**,
   daß die Acetylierung bei Temperaturen zwischen 85 und 115 °C vorgenommen wird.

3. Verfahren nach einem der Ansprüche 1 oder 2,
   **dadurch gekennzeichnet**,
   daß die Konzentration der zu acetylierenden α-Aminocarbonsäuren pro 1 Liter Essigsäure zwischen 0,5 und 2,5

Mol beträgt, wobei die Konzentration auf Standardbedingungen von Druck und Temperatur bezogen ist.

**4.** Verfahren nach einem der vorhergehenden Ansprüche,
**dadurch gekennzeichnet**,
daß die N-Acetyl-D,L-α-aminocarbonsäuren durch Eindampfen ohne weitere Isolierung oder Aufreinigung erhalten werden.

**5.** Verfahren nach einem der vorhergehenden Ansprüche,
**dadurch gekennzeichnet**,
daß das Acetylierungsgemisch zum Erhalt der N-Acetyl-D(L)-α-aminocarbonsäure mit einem Sambay-Verdampfer eingedampft wird.

**6.** Verfahren nach Anspruch 5,
**dadurch gekennzeichnet**,
daß die beim Einengen freiwerdende und abdestillierte Essigsäure wasserfrei ist und ohne weitere Aufreinigung zur Acetylierung rückgeführt wird.

**7.** Verfahren nach einem der vorherigen Ansprüche,
**dadurch gekennzeichnet**,
daß D,L-Methionin, -Valin, -Phenylalanin und/oder -Norvalin acetyliert werden.

**8.** Verfahren nach Anspruch 7,
**dadurch gekennzeichnet**,
daß D,L-Methionin acetyliert wird.

**9.** Verfahren nach Anspruch 8,
**dadurch gekennzeichnet**,
daß die Acetylierung des D,L-Methionin eine Stufe eines Verfahrens zur Gewinnung optisch aktiver α-Aminosäuren ist,
bei dem D,L-Methionin zunächst mit Essigsäure/Acetanhydrid N-acetyliert wird, das N-Acetyl-D,L-Methionin-Racemat enzymatisch gespalten wird, das L-Methionin unter Rückbehalt einer Mutterlauge abgetrennt wird und das nicht umgesetzte N-Acetyl-D(L)-Methionin der Mutterlauge nach Racemisierung als Rückführlösung zur enzymatischen Racemat-Spaltung rezykliert wird.

**Claims**

**1.** A process for the preparation of N-acetyl-D,L-α-aminocarboxylic acids from the corresponding D,L-α-aminocarboxylic acids by acetylation in acetic acid and isolation of the resulting N-acetyl-D,L-α-aminocarboxylic acids from the acetylation mixture, characterised in that acetylation is carried out with 1.0 mole to 1.1 mole of acetic anhydride per mole of D,L-α-amino acid to be acetylated at 60 °C to 150 °C in a concentration of $<$ 3 mole of α-aminocarboxylic acid per 1 litre of acetic acid, the concentration being based on standard conditions of pressure and temperature, and the N-acetyl-D,L-α-aminocarboxylic acid is obtained by evaporation as a melt or a solid.

**2.** A process according to claim 1, characterised in that acetylation is carried out at temperatures from 85 °C to 115 °C.

**3.** A process according to one of claims 1 or 2, characterised in that the concentration of the α-aminocarboxylic acids to be acetylated per 1 litre of acetic acid is from 0.5 mole to 2.5 mole, the concentration being based on standard conditions of pressure and temperature.

**4.** A process according to one of the preceding claims, characterised in that the N-acetyl-D,L-α-aminocarboxylic acids are obtained by evaporation without further isolation or purification.

**5.** A process according to one of the preceding claims, characterised in that the acetylation mixture is evaporated with a Sambay evaporator to obtain the N-acetyl-D(L)-α-aminocarboxylic acid.

**6.** A process according to claim 5, characterised in that the acetic acid released and distilled during concentration is water-free and is returned to acetylation without further purification.

7. A process according to one of the preceding claims, characterised in that D,L-methionine, -valine, -phenylalanine and/or -norvaline are acetylated.

8. A process according to claim 7, characterised in that D,L-methionine is acetylated.

9. A process according to claim 8, characterised in that the acetylation of D,L-methionine is a step of a process for obtaining optically active α-amino acids wherein D,L-methionine is initially N-acetylated with acetic acid/acetic anhydride, the N-acetyl-D,L-methionine racemate is enzymatically split, the L-methionine is separated whilst retaining a mother liquor, and the unreacted N-acetyl-D(L)-methionine of the mother liquor, after racemisation, is recycled as a recycle solution to enzymatic racemate splitting.

**Revendications**

1. Procédé de préparation d'acides N-acétyl-D,L-a-aminocarboxyliques à partir des acides D,L-α-aminocarboxyliques correspondants par acétylation dans l'acide acétique et isolement des acides N-acétyl-D,L-α-aminocarboxyliques du mélange d'acétylation,
caractérisé en ce que
l'acétylation est effectuée avec 1,0-1.1 mol d'anhydride acétique par mol d'acide DL-α-aminé à acétyler à 60-150°C à une concentration de < 3 mol d'acide α-aminocarboxylique pour 1 1 d'acide acétique, la concentration étant rapportée à des conditions standard de pression et de température,
et l'acide N-acétyl-D,L-a-aminocarboxylique est obtenu par concentration par évaporation sous forme de produit de fusion ou de matière solide.

2. Procédé selon la revendication 1,
caractérisé en ce que
l'acétylation est entreprise à des températures comprises entre 85 et 115°C.

3. Procédé selon l'une des revendications 1 ou 2,
caractérisé en ce que
la concentration de l'acide α-aminocarboxylique à acétyler par litre d'acide acétique se situe entre 0,5 et 2,5 mol, la concentration étant rapportée aux conditions standard de pression et de température.

4. Procédé selon l'une quelconque des revendications précédentes,
caractérisé en ce que
les acides N-acétyl-D,L-α-aminocarboxyliques sont obtenus par concentration par évaporation sans isolement ou purification supplémentaire.

5. Procédé selon l'une quelconque des revendications précédentes,
caractérisé en ce que
le mélange d'acétylation en vue de l'obtention de l'acide N-acétyl-D(L)-α-aminocarboxylique est concentré par distillation à l'aide d'un évaporateur Sambay.

6. Procédé selon la revendication 5,
caractérisé en ce que
l'acide acétique qui se libère lors de la concentration et qui est séparé par distillation, est anhydre et est recyclé sans purification supplémentaire en vue de l'acétylation.

7. Procédé selon l'une quelconque des revendications précédentes,
caractérisé en ce qu'
on acétyle la D,L-méthionine, D,L-valine, D,L-phénylalanine, et/ou la D,L-norvaline.

8. Procédé selon la revendication 7,
caractérisé en ce qu'
on acétyle la D,L-méthionine.

9. Procédé selon la revendication 8,
caractérisé en ce que

l'acétylation de la D,L-méthionine est une étape d'un procédé de production d'acides α-aminés optiquement actifs, dans lequel la D,L-méthionine est N-acétylée en premier lieu avec le mélange acide acétique/anhydride acétique, le racémate de N-acetyl-D,L-méthionine est clivé par voie enzymatique, la L-méthionine est séparée tout en conservant une liqueur mère, et la N-acétyl-D(L)-méthionine non transformée de la liqueur mère est recyclée après racémisation en tant que solution de récupération, en vue du clivage du racémate par vole enzymatique.